# FASCICULE DE BREVET EUROPEEN

(11) **EP 0 811 591 B1**
(45) Date de publication et mention de la délivrance du brevet: **28.03.2001**
(21) Numéro de dépôt: 97401145.4
(22) Date de dépôt: 26.05.1997
(51) Int. Cl.: C07C 17/395, C07C 19/08

(54) **Procédé de purification d'hydrofluorocarbures saturés**
Verfahren zur Reinigung von gesättigten Fluorkohlenwasserstoffen
Process for the purification of saturated hydrofluorocarbons

(30) Priorité: 06.06.1996 FR 9606992
(43) Date de publication de la demande: 10.12.1997
(73) Titulaire: Atofina, 92800 Puteaux (FR)
(72) Inventeur: Sage, Jean-Marc, 69600 Oullins (FR); Lacroix, Eric, 69480 Amberieux D'Azergues (FR)
(74) Mandataire: Leboulenger, Jean

(56) Documents cités:
- US-A- 3 004 908
- DATABASE WPI Section Ch, Week 9306 Derwent Publications Ltd., London, GB; Class A60, AN 93-049555 XP002023275 & JP 05 000 972 A (TECHNOLOGY AG KK) , 8 Janvier 1993

## Description

La présente invention concerne le domaine des hydrocarbures halogénés et a plus particulièrement pour objet la purification des hydrofluorocarbures, connus dans le métier sous la désignation HFC. Ces composés tels que par exemple le pentafluoroéthane (R-125) et le 1,1,1,2-tétrafluoroéthane (R-134a) sont actuellement développés afin de remplacer les CFC (chlorofluorocarbures) qui, comme par exemple le dichlorodifluorométhane (R-12), sont suspectés de contribuer à l'appauvrissement de la couche d'ozone.

La fabrication d'un hydrofluorocarbure, par exemple par fluoration d'un composé en C₂ au moyen de l'acide fluorhydrique, requiert bien sûr des catalyseurs et des conditions opératoires appropriés, mais en général il faut aussi considérer la purification du produit final. En effet, bien qu'ils conduisent à des rendements et sélectivités élevés, les procédés et catalyseurs mis en oeuvre lors de la synthèse des composés HFC génèrent des impuretés qui sont souvent difficiles à séparer ou éliminer par les moyens classiques tels que la distillation ou la dissolution sélective de ces impuretés dans un solvant approprié. De plus, même présentes en quantité peu importante, certaines de ces impuretés doivent être éliminées en raison de leur toxicité. Parmi ces impuretés, on peut citer plus particulièrement les dérivés oléfiniques et notamment ceux contenant deux atomes de carbone et des proportions variables d'atomes d'hydrogène, de fluor et/ou de chlore.

Différentes méthodes permettant d'éliminer les impuretés oléfiniques ou d'en réduire la teneur sont déjà décrites dans l'art antérieur. Ainsi, l'oléfine 2-chloro-1,1-difluoroéthylène (R-1122) contenue dans un R-134a peut être éliminée par passage sur un charbon actif (brevet EP 389 334) ou par traitement avec un hydrure métallique dans le tétrahydrofuranne (brevet EP 508 631).

Pour éliminer les oléfines CF₃-CCl=CCl-CF₃, CF₃-CCl=CH-CF₃, CCl₂=CF₂ et CF₂=CFCl présentes en impuretés dans un 1,1-dichloro-2,2,2-trifluoroéthane (R-123), il a été proposé dans le brevet EP 357 328 de traiter le R-123 par une solution aqueuse basique de permanganate de potassium.

Dans le brevet EP 370 688, la teneur en impuretés oléfiniques d'un composé HFC ou HCFC (hydrochlorofluorocarbure) est réduite par passage sur un lit d'oxyde métallique constitué d'au moins un oxyde de Cu(ll), Co(ll), Ag(I) ou Mn(II) à une température comprise entre 20 et 300°C.

Le brevet US 5 001 287 a préconisé l'hydrogénation catalytique des impuretés oléfiniques par passage du composé HFC impur sur un catalyseur tel que Pd/C en présence d'hydrogène.

Dans le brevet EP 548 744 on a proposé de traiter le composé HFC ou HCFC à purifier au moyen de fluor à une température de l'ordre de -80 à -40°C. Cependant, l'emploi de fluor sous sa forme diatomique F₂ est industriellement très difficile en raison des dangers liés à la très grande réactivité du fluor qui oblige à le diluer très fortement dans de l'azote.

L'élimination d'une oléfine peut également être effectuée par photo-oxydation. Cette méthode dans laquelle le produit à purifier est irradié avec un rayonnement de type UV en présence d'oxygène est décrite par exemple dans le brevet FR 2 698 094 pour la purification d'un 1,1-dichloro-1-fluoroéthane (R-141b).

Dans le brevet US 5 430 205 on a proposé d'éliminer les impuretés oléfiniques d'un R-134a en faisant passer un mélange gazeux de R-134a brut, d'acide fluorhydrique et d'oxygène ou d'air sur un catalyseur de fluoration à une température comprise entre 200 et 300°C. Toutefois, dans ces conditions, le R-134a et son précurseur le R-133a (1-chloro-2,2,2-trifluoroéthane) peuvent subir une combustion partielle conduisant à une perte de productivité.

Il est également connu (JP 06 080 592) d'éliminer les oléfines halogénées contenues dans un HCFC en le faisant passer en phase gaz sur un charbon actif en présence d'oxygène à une température comprise entre 80 et 300°C. Cependant, lorsque les impuretés sont présentes en quantités importantes, il est nécessaire de procéder à de fréquentes régénérations du charbon actif.

Selon la demande de brevet JP 05 000 972 on utilise l'ozone pour purifier des HCFC ou des HFC. Cependant, l'ozone présente l'inconvénient d'être un gaz toxique qui, même à des teneurs relativement faibles dans l'atmosphère ambiante, peut induire des problèmes pour la santé humaine.

Il a maintenant été trouvé que l'introduction d'oxygène (pur ou sous forme d'air) dans un composé HFC permet d'en éliminer de façon pratiquement totale les impuretés oléfiniques, notamment les oléfines en C₂ et plus particulièrement le chlorotrifluoroéthylène. Cette élimination ne nécessite pas la présence d'un catalyseur ou d'un charbon actif. Elle se produit à une température modérée et peut être réalisée en phase liquide, ce qui est particulièrement intéressant du point de vue industriel. Ainsi, par simple addition d'une quantité appropriée d'oxygène, une oléfine comme le chlorotrifluoroéthylène (R-1113 ou CTFE) peut être complètement éliminée d'un HFC comme le R-125.

L'invention a donc pour objet un procédé de purification d'un composé HFC contenant au moins une impureté oléfinique, caractérisé en ce qu'il comprend une étape consistant à mettre en contact le dit HFC impur avec de l'oxygène, en l'absence de catalyseur ou de charbon actifs, sous réserve que ladite étape ne consiste pas en la mise en contact du 1,1,1,2-tétrafluoroéthane avec un mélange d'oxygène et d'ozone comprenant 2% an poids d'ozone.

Dans un composé HFC à purifier, la teneur pondérale en oléfine(s) est généralement comprise entre 10 et 10000 ppm, le plus souvent entre 10 et 1000 ppm. Bien qu'il vise plus particulièrement la purification des fluoroéthanes contenant de 2 à 5 atomes de fluor et préférentiellement celle du R-125, le traitement à l'oxygène selon l'invention peut aussi être appliqué pour purifier un composé HFC contenant 3 ou plus de 3 atomes de carbone.

La quantité d'oxygène à ajouter au composé HFC à purifier peut aller de 10 à 10000 ppm en poids et est de préférence comprise entre 500 et 5000 ppm. Elle dépend évidemment de la teneur en oléfine(s) du produit à purifier et des conditions de température et de pression mises en oeuvre. D'une façon générale, il convient d'utiliser une quantité d'oxygène telle que le rapport molaire oxygène/oléfine(s) soit compris entre 1 et 1000, de préférence entre 1 et 200 et, plus particulièrement, entre 1 et 50.

L'oxygène peut être introduit dans le composé HFC à purifier de façon continue ou discontinue, soit sous forme d'oxygène pur, soit dilué dans un autre gaz tel que l'azote, par exemple sous forme d'air.

La durée du traitement peut varier dans de larges limites (de quelques minutes à plusieurs jours). Elle dépend en effet de la teneur en oléfine(s) à éliminer, de la quantité d'oxygène ajouté et des conditions de température et de pression mises en oeuvre. L'opération peut être réalisée à une température allant de -40 à +200°C, de préférence comprise entre 10 et 150°C et, avantageusement, autour de la température ambiante. La pression peut aller de 10 à 10000 kPa, mais est de préférence comprise entre 500 et 5000 kPa.

Le traitement selon l'invention peut être mis en oeuvre en phase liquide ou en phase gaz, mais d'un point de vue pratique il est préférable d'opérer en phase liquide.

Après le traitement selon l'invention, le composé HFC débarrassé de ses impuretés oléfiniques peut évidemment être soumis à une autre étape de purification pour éliminer les autres impuretés initialement présentes et/ou les produits générés lors du traitement à l'oxygène selon l'invention tels que des résidus d'HF, HCl, CF₃COOH, COF₂, COFCl ou COCl₂. Cette autre étape peut être par exemple un lavage par une solution aqueuse alcaline ou un passage sur un charbon actif, en particulier un charbon actif alcalin.

Les exemples suivants illustrent l'invention sans la limiter. Les ppm sont exprimées en poids sauf mention contraire.

### EXEMPLE 1 (Comparatif)

Dans un tube en verre d'environ 8 ml de volume, on introduit en absence d'air par piégeage à l'azote liquide 2,0g de pentafluoroéthane (R-125) contenant 450 ppm de chlorotrifluoroéthylène (R-1113), 10000 ppm de chloropentafluoroéthane (R-115), 285 ppm de 1,1,1-trifluoroéthane (R-143a) et 100 ppm 1-chloro-1,2,2,2-tétrafluoroéthane (R-124). Le tube maintenu sous vide et à la température de l'azote liquide est ensuite scellé, puis introduit dans un appareil chauffant à l'abri de la lumière et agité par balancement. La température est portée à 80°C pendant 48 heures.

Au bout de ce laps de temps, le tube scellé est refroidi à la température de l'azote liquide et mis en communication avec une bouteille en acier (volume : 20 ml) préalablement mise sous vide et maintenue à la température de l'azote liquide. On casse ensuite le haut du tube et on réchauffe doucement le tube jusqu'à la température ambiante pour récupérer les gaz par piégeage dans l'éprouvette métallique.

On récupère ainsi dans l'éprouvette 2 g de gaz dont l'analyse CPV montre que la composition du R-125 est restée pratiquement inchangée.

### EXEMPLE 2

On opère comme à l'exemple 1 avec le même R-125, mais en ajoutant aux 2 g de R-125 2700 ppm d'oxygène, soit 1 % en volume par rapport au R-125 gazeux.

Après 48 heures de réaction, l'analyse du R-125 récupéré montre que la teneur en R-1113 est tombée en-dessous de 3 ppm, les teneurs initiales en R-115, R-143a et R-124 étant restées inchangées.

### EXEMPLE 3

A un R-125 impur contenant 13 ppm de R-1113, 20ppm de R-143a et 675 ppm de R-134a, on ajoute 0,5 % en volume d'air (soit 270 ppm d'oxygène), puis on le stocke dans un récipient en tôle zinguée pendant 30 jours à température ambiante (10 à 20°C) et à une pression absolue de 1200 kPa.

Au bout de ce laps de temps, l'analyse du produit montre que sa teneur en R-1113 est devenue inférieure à 1 ppm, les teneurs en R-143a et R-134a n'ayant pratiquement pas changé.

Le produit peut ensuite être passé sur un charbon actif pour le débarrasser des traces d'acidité formées lors du traitement.

## Revendications

1. Procédé de purification d'un hydrofluorocarbure (HFC) contenant au moins une impureté oléfinique, caractérisé en ce qu'il comprend une étape consistant à mettre en contact ledit HFC impur avec de l'oxygène, en l'absence de catalyseur ou de charbon actif, sous réserve que ladite étape ne consiste pas en la mise en contact du 1,1,1,2-tétrafluoroéthane avec un mélange d'oxygène et d'ozone comprenant 2% en poids d'ozone.

2. Procédé selon la revendication 1 dans lequel l'impureté oléfinique est une oléfine en C₂, en particulier le chlorotrifluoroéthylène.

3. Procédé selon la revendication 1 ou 2 dans lequel le rapport molaire : oxygène/oléfine(s) est compris entre 1 et 1000, de préférence entre 1 et 200, et plus particulièrement entre 1 et 50.

4. Procédé selon l'une des revendications 1 à 3, dans lequel le traitement est effectué à une température allant de -40 à +200°C, de préférence comprise entre 10 et 150°C et, avantageusement, autour de la température ambiante.

5. Procédé selon l'une des revendications 1 à 4 dans lequel le traitement est effectué à une pression comprise entre 10 et 10000 kPa, de préférence entre 500 et 5000 kPa.

6. Procédé selon l'une des revendications 1 à 5 dans lequel on opère en phase liquide.

7. Procédé selon l'une des revendications 1 à 6 dans lequel l'oxygène est introduit sous forme d'air.

8. Application du procédé selon l'une des revendications 1 à 7 à la purification d'un fluoroéthane contenant de 2 à 5 atomes de fluor et préférentiellement à celle du pentafluoroéthane

9. Application du procédé selon l'une des revendications 1 à 7 pour l'élimination du chlorotrifluoroéthylène présent dans un hydrofluorocarbure, en particulier dans un fluoroéthane contenant de 2 à 5 atomes de fluor.

10. Application du procédé selon l'une des revendications 1 à 7 pour l'élimination du chlorotrifluoroéthylène présent dans un pentafluoroéthane.

## Patentansprüche

1. Verfahren zur Reinigung eines Fluorkohlenwasserstoffs (FKW), der mindestens eine olefinische Verunreinigung enthält, dadurch gekennzeichnet, daß es einen Schritt enthält, der darin besteht, den genannten unreinen FKW mit Sauerstoff in Abwesenheit eines Katalysators oder von Aktivkohle zusammenzubringen, vorbehaltlich daß der genannte Schritt nicht darin besteht, 1,1,1,2-Tetrafluorethan mit einem Gemisch aus Sauerstoff und Ozon mit 2 Gew.-% Ozon zusammenzubringen.

2. Verfahren nach Anspruch 1, in dem die olephinische Verunreinigung ein C₂-Olefin, insbesondere Chlortrifluorethylen, ist.

3. Verfahren nach Anspruch 1 oder 2, in dem das Molverhältnis Sauerstoff/ Olefin(en) zwischen 1 und 1.000, vorzugsweise zwischen 1 und 200 und noch spezieller zwischen 1 und 50, liegt.

4. Verfahren nach einem der Ansprüche 1 bis 3, in dem die Behandlung bei einer Temperatur von -40 bis +200 °C, vorzugsweise zwischen 10 und 150 °C und vorteilhafterweise nahe der Umgebungstemperatur, durchgeführt wird.

5. Verfahren nach einem der Ansprüche 1 bis 4, in dem die Behandlung bei einem Druck zwischen 10 und 10.000 kPa, vorzugsweise zwischen 500 und 5.000 kPa, durchgeführt wird.

6. Verfahren nach einem der Ansprüche 1 bis 5, in dem man in flüssiger Phase arbeitet.

7. Verfahren nach einem der Ansprüche 1 bis 6, in dem der Sauerstoff in Form von Luft zugegeben wird.

8. Verwendung des Verfahrens nach einem der Ansprüche 1 bis 7 zur Reinigung eines Fluorethans mit 2 bis 5 Fluoratomen und vorzugsweise von Pentafluorethan.

9. Verwendung des Verfahrens nach einem der Ansprüche 1 bis 7 zur Entfernung des in einem Fluorkohlenwasserstoff, insbesondere in einem Fluorethan mit 2 bis 5 Fluoratomen, vorliegenden Chlortrifluorethylens.

10. Verwendung des Verfahrens nach einem der Ansprüche 1 bis 7 zur Entfernung des in Pentafluorethan vorliegenden Chlortrifluorethylens.

## Claims

1. Process for the purification of a hydrofluorocarbon (HFC) containing at least one olefinic impurity, characterized in that it comprises a stage which consists in bringing the said impure HFC into contact with oxygen, in the absence of catalyst or of active charcoal, with the proviso that the said stage does not consist in bringing 1,1,1,2-tetrafluoroethane into contact with a mixture of oxygen and ozone comprising 2% by weight of ozone.

2. Process according to Claim 1, in which the olefinic impurity is a C₂ olefin, in particular chlorotrifluoroethylene.

3. Process according to Claim 1 or 2, in which the molar ratio:oxygen/olefin(s) is between 1 and 1000, preferably between 1 and 200 and more particularly between 1 and 50.

4. Process according to one of Claims 1 to 3, in which the treatment is carried out at a temperature ranging from -40 to +200°C, preferably between 10 and 150°C and, advantageously, around room temperature.

5. Process according to one of Claims 1 to 4, in which the treatment is carried out at a pressure of between 10 and 10,000 kPa, preferably between 500 and 5000 kPa.

6. Process according to one of Claims 1 to 5, in which the process is carried out in the liquid phase.

7. Process according to one of Claims 1 to 6, in which the oxygen is introduced in the form of air.

8. Application of the process according to one of Claims 1 to 7 to the purification of a fluoroethane containing from 2 to 5 fluorine atoms and preferentially to that of pentafluoroethane.

9. Application of the process according to one of Claims 1 to 7 for the removal of chlorotrifluoroethylene present in a hydrofluorocarbon, in particular in a fluoroethane containing from 2 to 5 fluorine atoms.

10. Application of the process according to one of Claims 1 to 7 for the removal of chlorotrifluoroethylene present in a pentafluoroethane.
